# EUROPEAN PATENT APPLICATION

(11) **EP 4 397 662 A1**
(43) Date of publication of application: **10.07.2024**
(21) Application number: 22864071.0
(22) Date of filing: 15.07.2022
(51) Int. Cl.: C07D 487/04, B01D 11/02, C07C 17/35

(54) **METHOD FOR RECOVERING CATION COMPONENT OF IONIC LIQUID AND METHOD FOR REGENERATING IONIC LIQUID**

(30) Priority: 01.09.2021 JP 2021142173
(71) Applicant: Nisshinbo Holdings Inc., Tokyo 103-8650 (JP)
(72) Inventor: MASUDA Gen, Chiba-city, Chiba 267-0056 (JP)
(74) Representative: Mewburn Ellis LLP
(86) International application number: PCT/JP2022/027789
(87) International publication number: WO 2023/032483

(57) **Abstract**

A method for recovering a cation component of a neutralized-salt type ionic liquid, characterized by comprising a first step, in which an aqueous solution of the neutralized-salt type ionic liquid is mixed with a water-incompatible organic solvent to prepare a liquid separating into two phases, i.e., an aqueous phase and an organic phase, a second step, in which the liquid separating into two phases is mixed with a water-soluble strong alkali so that the amount of the strong alkali is 20% by mass or larger with respect to the water contained in the aqueous phase, thereby regenerating the cations of the neutralized-salt type ionic liquid present in the aqueous phase, into a neutral substance and moving the neutral substance from the aqueous phase to the organic phase, and a third step, in which the organic phase containing the neutral substance is recovered by a liquid-separating operation. By this method, the cation component of an expensive ionic liquid can be easily recovered at low cost as compared with conventional methods.

## Description

### TECHNICAL FIELD

The present invention relates to a method for recovering a cation component of an ionic liquid and a method for regenerating an ionic liquid.

### BACKGROUND ART

Cellulose expected as a biofuel is difficult to dissolve or extract by using water or an organic solvent, and there is a problem in that existing techniques for treating cellulose with a strong acid under a high temperature and a high pressure require a large amount of energy.

Therefore, in recent years, techniques such as isolation/purification, decomposition, and modification reaction of cellulose using an ionic liquid capable of dissolving cellulose under mild conditions have attracted attention.

On the other hand, since the ionic liquid is an expensive substance, it is also important to develop a technique for recovering the ionic liquid after being used for cellulose dissolution or the like.

In this regard, Non-Patent Document 1 discloses a method in which a neutralized-salt type ionic liquid solution of cellulose is put into water to recover cellulose, the aqueous solution of the neutralized-salt type ionic liquid from which cellulose has been recovered is heated to distill off water, and the neutralized-salt type ionic liquid is recovered as a residual component.

In this method, there is a problem in that a cation component is partially decomposed at the time of distilling water by heating and the recovery rate is lowered, and there is also a problem in that, although a large amount of energy is consumed for distilling a large amount of water, the amount of ionic liquid that can be recovered is small and the energy efficiency is very poor.

### PRIOR ART DOCUMENTS

### NON-PATENT DOCUMENTS

Non-Patent Document 1: RCS Advance, 2015, Vol. 5, page 69728

### SUMMARY OF INVENTION

### TECHNICAL PROBLEM

The present invention has been made in view of such circumstances, and an object of the present invention is to provide a method for recovering a cation component of an ionic liquid by which the cation component of the ionic liquid that is expensive can be recovered by an inexpensive and simple method as compared with a conventional method, and a method for regenerating an ionic liquid by which the ionic liquid can be regenerated only by adding an anion component to a recovered cation component.

### SOLUTION TO PROBLEM

The present inventor has conducted intensive studies in order to achieve the above object, and as a result, has found that the above object can be achieved by the following method using a two-phase separated liquid prepared by mixing an aqueous solution of a neutralized-salt type ionic liquid with a water-insoluble organic solvent, thereby completing the present invention.

That is, the present invention provides as follows:
1. A method for recovering a cation component of a neutralized-salt type ionic liquid, the recovery method including:
   a first step of mixing an aqueous solution of a neutralized-salt type ionic liquid with an organic solvent incompatible with water to prepare a two-phase separated liquid of an aqueous phase and an organic phase;
   a second step of mixing the two-phase separated liquid with a strong alkali soluble in water so that an amount of the strong alkali is 20% or more with respect to water in the aqueous phase in terms of weight ratio to regenerate a cation of the neutralized-salt type ionic liquid present in the aqueous phase into a neutral substance and move the neutral substance from the aqueous phase to the organic phase; and
   a third step of recovering the organic phase containing the neutral substance by a liquid-separating operation;
2. The recovery method according to 1, including:
   a fourth step of mixing the organic phase recovered in the third step with an aqueous solution of a new neutralized-salt type ionic liquid; and
   a fifth step of repeating the second step and the third step again after the fourth step to recover the organic phase in which a content of the neutral substance is increased;
3. The recovery method according to 1 or 2, wherein in the second step, the two-phase separated liquid and the strong alkali are mixed so that the amount of the strong alkali is 25% or more with respect to the water in terms of weight ratio;
4. The recovery method according to any one of 1 to 3, wherein in the second step, a reaction liquid temperature during mixing the two-phase separated liquid with the strong alkali is 20°C or lower;
5. The recovery method according to 4, wherein the reaction liquid temperature is 10°C or lower;
6. The recovery method according to any one of 1 to 5, wherein in the third step, the recovery of the organic phase is started within 1 hour from completion of mixing in the second step;
7. The recovery method according to 6, wherein in the third step, the recovery of the organic phase is started within 30 minutes from completion of mixing in the second step;
8. The recovery method according to any one of 1 to 7, wherein the strong alkali is sodium hydroxide or potassium hydroxide;
9. The recovery method according to any one of 1 to 8, wherein the cation of the neutralized-salt type ionic liquid has the following Formula (1) or (2): 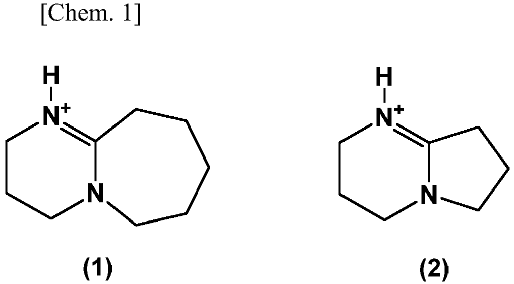
10. The recovery method according to any one of 1 to 9, wherein an anion of the neutralized-salt type ionic liquid is an anion of an organic acid;
11. The recovery method according to 10, wherein the organic acid is acetic acid;
12. The recovery method according to any one of 1 to 11, wherein the neutralized-salt type ionic liquid has a cellulose dissolving ability;
13. The recovery method according to 12, wherein the aqueous solution of the neutralized-salt type ionic liquid used in the first step is a coagulation liquid obtained after cellulose regeneration from a neutralized-salt type ionic liquid solution of cellulose;
14. A method for regenerating a neutralized-salt type ionic liquid, the regeneration method including mixing the organic phase containing the neutral substance recovered by the recovery method according to any one of 1 to 13 with an acid providing an anion component constituting the original neutralized-salt type ionic liquid and then removing the organic solvent to regenerate a neutralized-salt type ionic liquid;
15. A method for isolating a neutral substance, the isolation method including distilling the organic phase containing the neutral substance recovered by the recovery method according to any one of 1 to 13 to remove the organic solvent, and isolating the neutral substance; and
16. A method for regenerating a neutralized-salt type ionic liquid, the regeneration method including mixing the neutral substance obtained by the isolation method according to 15 with an acid providing an anion component constituting the original neutralized-salt type ionic liquid to regenerate a neutralized-salt type ionic liquid.

### ADVANTAGEOUS EFFECTS OF INVENTION

According to the present invention, it is possible to recover a cation component of an ionic liquid that is expensive by an inexpensive and simple method as compared with a conventional method. This method is an environmentally friendly method because it does not consume a large amount of energy. An ionic liquid can be regenerated only by adding an anion component to a recovered cation component.

By using the recovery method of the present invention for recovery of an ionic liquid after cellulose spinning, it is possible to reduce the cost by recycling the ionic liquid.

### DESCRIPTION OF EMBODIMENTS

Hereinafter, the present invention is described in more detail.

A method for recovering a cation component of a neutralized-salt type ionic liquid according to the present invention includes a first step of mixing an aqueous solution of a neutralized-salt type ionic liquid with an organic solvent incompatible with water to prepare a two-phase separated liquid of an aqueous phase and an organic phase, a second step of mixing the two-phase separated liquid obtained in the first step with a strong alkali soluble in water to regenerate a cation of the neutralized-salt type ionic liquid present in the aqueous phase into a neutral substance and move the neutral substance from the aqueous phase to the organic phase, and a third step of recovering the organic phase containing the neutral substance by a liquid-separating operation.

In the present invention, "incompatible with water" in the "organic solvent incompatible with water" means that two phases are separated if water and the organic solvent are mixed at a volume ratio of 1 : 1, and the strong alkali means that the degree of ionization in the aqueous solution is close to 1 and the basic dissociation constant is about pK_{b} < 0 (K_{b} > 1).

### [Neutralized-salt type ionic liquid]

The neutralized-salt type ionic liquid is an ionic liquid composed of a salt obtained by an acid-base neutralization reaction (see "Ionic liquids-The Front and Future of Material Development-, pp. 19 to 21, published by CMC Publishing Co., LTD. (2003)"), and refers to one having a cation formed by addition of a proton.

The neutralized-salt type ionic liquid to be recovered in the present invention may be an ionic liquid composed of a salt obtained by an acid-base neutralization reaction, and cations and anions constituting the ionic liquid are not particularly limited, but an ionic liquid having a cellulose dissolving ability is preferable, and one having a cation having the following Formula (1) or (2) is more preferable.

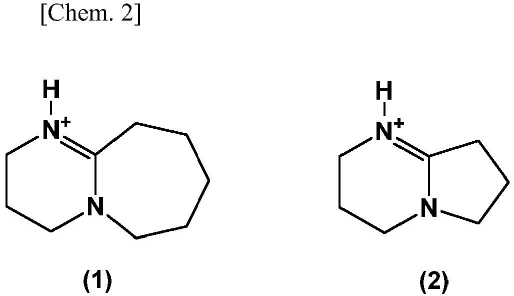

The anion is preferably an anion of an organic acid, and examples of the organic acid include a carboxylic acid such as formic acid or acetic acid; and a sulfonic acid such as methanesulfonic acid or p-toluenesulfonic acid, but a carboxylic acid is preferable, and acetic acid is more preferable.

### [First step]

The first step is a step of mixing an aqueous solution of a neutralized-salt type ionic liquid (hereinafter, it may be simply referred to as "aqueous solution") with an organic solvent incompatible with water (hereinafter, it may be simply referred to as "organic solvent") to prepare a two-phase separated liquid of an aqueous phase and an organic phase.

In the first step, the method of mixing the aqueous solution of the neutralized-salt type ionic liquid with the organic solvent is not particularly limited, and the organic solvent may be added to the aqueous solution, or the aqueous solution may be added to the organic solvent, but the method of adding the organic solvent to the aqueous solution is preferable. Two-phase separation may be performed after stirring during mixing and/or after mixing.

The temperature during mixing may be equal to or lower than the boiling point of water, but is preferably 30°C or lower, more preferably 20°C or lower, and still more preferably 10°C or lower.

The amount of the organic solvent used is not particularly limited as long as the two-phase separated liquid can be prepared, but is preferably 10 : 1 to 1 : 10, more preferably 5 : 1 to 1 : 5, and still more preferably 2 : 1 to 1 : 2 with respect to the aqueous solution in terms of volume ratio.

The organic solvent used in the first step preferably has at least a dissolving ability of the neutral substance generated in the second step, and further preferably has no dissolving ability of the neutralized-salt type ionic liquid regenerated from the recovered neutral substance.

Specific examples thereof are not necessarily limited because they depend on the type of the neutralized-salt type ionic liquid (neutral substance), but include aliphatic hydrocarbon-based solvents (such as pentane, n-hexane, n-octane, n-decane, and decalin), aromatic hydrocarbon-based solvents (such as benzene, nitrobenzene, toluene, o-xylene, m-xylene, p-xylene, and mesitylene), halogenated aromatic hydrocarbon-based solvents (such as chlorobenzene, bromobenzene, o-dichlorobenzene, m-dichlorobenzene, and p-dichlorobenzene), ether-based solvents (such as diethyl ether, diisopropyl ether, t-butyl methyl ether, tetrahydrofuran, dioxane, 1,2-dimethoxyethane, and 1,2-diethoxyethane), and ester-based solvents (such as ethyl acetate and butyl acetate), and these solvents may be used singly or in combination of two or more kinds thereof.

Among them, in the case of the neutralized-salt type ionic liquid having the cation having the above Formula (1) or Formula (2), an aliphatic hydrocarbon-based solvent and an aromatic hydrocarbon solvent are preferable, and toluene is more preferable.

As the aqueous solution of the neutralized-salt type ionic liquid used in the first step, a coagulation liquid obtained after cellulose regeneration from a neutralized-salt type ionic liquid solution of cellulose can be used, and this makes it possible to recover, regenerate, and reuse the ionic liquid which has been used for cellulose regeneration.

### [Second step]

The second step is a step of mixing the two-phase separated liquid obtained in the first step with a strong alkali soluble in water to regenerate a cation of the neutralized-salt type ionic liquid present in the aqueous phase into a neutral substance and move the neutral substance from the aqueous phase to the organic phase.

Examples of the strong alkali soluble in water used in the second step include hydroxides of alkali metals such as lithium hydroxide, sodium hydroxide, potassium hydroxide, rubidium hydroxide, and cesium hydroxide; and hydroxides of tetraalkylammonium such as tetramethylammonium hydroxide and tetraethylammonium hydroxide, but in consideration of cost, sodium hydroxide and potassium hydroxide are preferable, and sodium hydroxide is more preferable.

The method of mixing the two-phase separated liquid with the strong alkali is also not particularly limited, and the strong alkali may be added to the two-phase separated liquid, or the aqueous solution may be added to the aqueous solution, but the method of adding the strong alkali to the aqueous solution is preferable because heat is generated. In this case, the strong alkali in a solid (pellet) form may be used as it is or the strong alkali may be used as an aqueous solution. Stirring may be performed during mixing and/or after mixing.

The reaction liquid temperature at the time of mixing is preferably 20°C or lower, more preferably 15°C or lower, and still more preferably 10°C or lower in consideration of improving the recovery rate of a target product by suppressing generation of a decomposition product, and in order to maintain this temperature range, cooling may be performed from the outside using an ice bath, a refrigerant, or the like.

The amount of the strong alkali used is preferably 20% or more and more preferably 25 to 50% with respect to water in the aqueous phase constituting the two-phase separated liquid in terms of weight ratio, from the viewpoint of suppressing the decomposition of the neutral substance and further increasing the recovery rate.

### [Third step]

The third step is a step of recovering the organic phase containing the neutral substance after the second step by a liquid-separating operation.

The liquid separation method is not particularly limited, and may be performed by a known method.

The liquid separation is preferably performed as soon as possible after the second step, and in particular, the recovery of the organic phase is more preferably started within 1 hour from completion of mixing of the two-phase separated liquid with the strong alkali soluble in water in the second step, and still more preferably started within 30 minutes.

An organic solvent may be added to the aqueous phase after liquid separation, and the liquid separation/extraction operation may be repeated a plurality of times.

After the third step, a neutralized-salt type ionic liquid can be regenerated by mixing the recovered organic phase with an acid providing an anion component constituting the original neutralized-salt type ionic liquid and then removing the organic solvent. Also in this case, the acid may be added to the organic phase, or the organic phase may be added to the acid, but it is preferable to add the acid to the organic phase, and the temperature during mixing is preferably 30°C or lower, more preferably 20°C or lower, and still more preferably 10°C or lower, and in order to maintain this temperature range, cooling may be performed from the outside using an ice bath, a refrigerant, or the like.

A neutralized-salt type ionic liquid can also be regenerated by removing the organic solvent from the recovered organic phase by distillation to temporarily isolate the neutral substance, and mixing this neutral substance with an acid providing an anion component constituting the original neutralized-salt type ionic liquid. Also in this case, the acid may be added to the neutral substance, or the neutral substance may be added to the acid.

### [Fourth and fifth steps]

The recovery method of the present invention may include a fourth step of mixing the organic phase recovered in the third step with an aqueous solution of a new neutralized-salt type ionic liquid, and a fifth step of repeating the second step and the third step again after the fourth step to increase a content of the neutral substance in the organic phase and then recovering the organic phase.

In the mixing in the fourth step, the aqueous solution may be added to the organic phase, or the organic phase may be added to the aqueous solution, but it is preferable to add the aqueous solution to the recovered organic phase. An organic solvent may be added as necessary during mixing. The temperature during mixing is preferably 40°C or lower, more preferably 30°C or lower, and still more preferably 25°C or lower.

As described above, the method for recovering a cation component of an ionic liquid of the present invention is an inexpensive and simple method using an organic solvent and a strong alkali, and is a method that does not consume a large amount of energy. An ionic liquid can be regenerated by a simple method of adding an anion component to a recovered cation component.

### EXAMPLES

Hereinafter, the present invention is more specifically described with reference to Examples and Comparative Examples, but the present invention is not limited to the following Examples.

### [1] Recovery of DBU (neutral substance)

### [Example 1-1]

To 2.00 g of a neutralized-salt type ionic liquid DBUH·AcO having the following formula, 100 mL of ion-exchanged water was added, the mixture was stirred to completely dissolve the neutralized-salt type ionic liquid to thereby prepare an aqueous solution of the neutralized-salt type ionic liquid, and 100 mL of toluene was added to the aqueous solution to prepare a two-phase separated liquid (first step).

To the prepared two-phase separated liquid, 30.00 g of sodium hydroxide (pellet) under ice cooling (reaction liquid temperature: 10°C or lower), and the mixture was stirred for 20 minutes after the pellet was charged (second step). The sodium hydroxide was completely dissolved in the aqueous phase within 10 minutes after charging.

The two-phase separated liquid after the second step was placed in a separating funnel, and after confirming the two-phase separation, the liquid was quickly separated to recover the organic phase (third step). The recovery was started immediately after mixing the two-phase separated liquid with sodium hydroxide and stirring for 20 minutes.

Toluene was distilled off from the recovered organic phase under reduced pressure, and DBU was recovered (recovered amount: 1.48 g, recovery rate: 99%). As a result of ¹H-NMR measurement, it was confirmed that this DBU contained almost no decomposition product (hydrolysate of DBU, N-(3-aminopropyl)caprolactam, the same applies hereinafter) and had a trace amount or less. 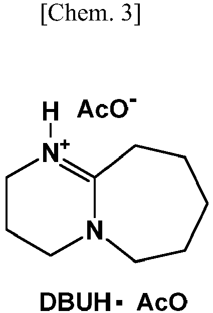 wherein Ac represents an acetyl group.

### [Example 1-2]

To 2.15 g of a neutralized-salt type ionic liquid DBUH·AcO, 100 mL of ion-exchanged water was added, the mixture was stirred to completely dissolve the neutralized-salt type ionic liquid to thereby prepare an aqueous solution of the neutralized-salt type ionic liquid, and 100 mL of toluene was added to the aqueous solution to prepare a two-phase separated liquid (first step).

To the prepared two-phase separated liquid, 10.05 g of sodium hydroxide (pellet) at room temperature, and the mixture was stirred for 20 minutes after the pellet was charged (second step). The sodium hydroxide was completely dissolved in the aqueous phase within 10 minutes after charging. At this time, the temperature of the reaction liquid was increased to 50°C or higher.

The two-phase separated liquid after the second step was placed in a separating funnel, and after confirming the two-phase separation, the liquid was quickly separated to recover the organic phase (third step). The recovery was started immediately after mixing the two-phase separated liquid with sodium hydroxide and stirring for 20 minutes.

Toluene was distilled off from the recovered organic phase under reduced pressure, and DBU was recovered (recovered amount: 0.81 g, recovery rate: 53%). As a result of ¹H-NMR measurement, it was recognized that this DBU contained about several% of a decomposition product.

### [Comparative Example 1-1]

To 2.10 g of a neutralized-salt type ionic liquid DBUH·AcO, 100 mL of ion-exchanged water was added, and the mixture was stirred to completely dissolve the neutralized-salt type ionic liquid. Thereafter, 30.03 g of sodium hydroxide (pellet) was added, and the mixture was stirred until the pellet was dissolved.

After 20 minutes from the charging of sodium hydroxide, 100 mL of toluene was added, the mixture was vigorously stirred for about 20 seconds, this reaction liquid was placed in a separating funnel, and after confirming the two-phase separation, the liquid was quickly separated to recover the organic phase. The sodium hydroxide was completely dissolved in the aqueous phase within 10 minutes after charging.

Toluene was distilled off from the recovered organic phase under reduced pressure, and the residue was recovered (recovered amount: 1.47 g, recovery rate: 98% if all residues are calculated as DBU). As a result of ¹H-NMR measurement, this residue was a mixture containing a hydrolysate of DBU at a ratio of DBU : the decomposition product of about 1 : 0.38 (approximate value from integral value).

From the results of Examples 1-1 and 1-2 and Comparative Example 1-1, it is found that DBU can be efficiently recovered by previously adding toluene to form a two-phase system and increasing the concentration of the strong alkali. In particular, as in Example 1-1, it is found that if sodium hydroxide is added at a low temperature, the decomposition of DBU is further suppressed, and DBU can be almost quantitatively recovered.

### [2] Regeneration of DBUH·AcO

### [Example 2-1]

To 4.11 g of a neutralized-salt type ionic liquid DBUH·AcO, 100 mL of ion-exchanged water was added, the mixture was stirred to completely dissolve the neutralized-salt type ionic liquid to thereby prepare an aqueous solution of the neutralized-salt type ionic liquid, and 100 mL of hexane was added to the aqueous solution to prepare a two-phase separated liquid (first step).

To the prepared two-phase separated liquid, 35.00 g of sodium hydroxide (pellet) under ice cooling (reaction liquid temperature: 10°C or lower), and the mixture was stirred for 20 minutes after the pellet was charged (second step). The sodium hydroxide was completely dissolved in the aqueous phase within 10 minutes after charging.

The two-phase separated liquid after the second step was placed in a separating funnel, and after confirming the two-phase separation, the liquid was quickly separated to recover the organic phase (third step). The recovery was started immediately after mixing the two-phase separated liquid with sodium hydroxide and stirring for 20 minutes.

If regenerated DBU and 1.16 g of equimolar acetic acid were added to the recovered organic phase, the liquid became instantaneously cloudy, transparent droplets adhered to the wall surface of the container, and DBUH·AcO separated from hexane into two phases was also confirmed at the lower portion. This two-phase separated liquid was directly subjected to an evaporator and subsequently a vacuum pump to remove hexane, thereby recovering DBUH·AcO (recovered amount: 3.83 g, recovery rate: 93%). As a result of ¹H-NMR measurement, it was confirmed that this DBUH·AcO contained almost no impurities and had a trace amount or less.

The liquid-separating operation was not carried out this time since there was a possibility of yield reduction if considering the loss in a small scale, but from the aspect of the two-phase separation, it was found that DBUH·AcO can be sufficiently recovered from hexane by the liquid-separating operation at the time of scale-up.

### [Example 2-2]

To 2.00 g of a neutralized-salt type ionic liquid DBUH·AcO, 100 mL of ion-exchanged water was added, the mixture was stirred to completely dissolve the neutralized-salt type ionic liquid to thereby prepare an aqueous solution of the neutralized-salt type ionic liquid, and 100 mL of toluene was added to the aqueous solution to prepare a two-phase separated liquid (first step).

To the prepared two-phase separated liquid, 30.06 g of potassium hydroxide (pellet) under ice cooling (reaction liquid temperature: 10°C or lower), and the mixture was stirred for 10 minutes after the pellet was charged (second step). The potassium hydroxide was completely dissolved in the aqueous phase within 5 minutes after charging.

The two-phase separated liquid after the second step was placed in a separating funnel, and after confirming the two-phase separation, the liquid was quickly separated to recover the organic phase (third step). The recovery was started immediately after mixing the two-phase separated liquid with potassium hydroxide and stirring for 10 minutes.

If regenerated DBU and 0.54 g of equimolar acetic acid were added to the recovered organic phase, the liquid became instantaneously cloudy and then became a hazy solution with a haze. This reaction liquid was directly subjected to an evaporator and subsequently a vacuum pump to remove hexane, thereby recovering DBUH·AcO (recovered amount: 1.96 g, recovery rate: 98%). As a result of ¹H-NMR measurement, it was confirmed that this DBUH·AcO contained almost no impurities and had a trace amount or less.

From the results of Examples 2-1 and 2-2, it is found that a neutralized-salt type ionic liquid can be regenerated in a high yield by using the recovery method and the regeneration method of the present invention. It is found that two-phase separation is performed by a combination of an organic solvent to be used and an amine, and a regenerated component can be recovered by a liquid-separating operation simpler than solvent distillation.

## Claims

1. A method for recovering a cation component of a neutralized-salt type ionic liquid, the recovery method comprising:
a first step of mixing an aqueous solution of a neutralized-salt type ionic liquid with an organic solvent incompatible with water to prepare a two-phase separated liquid of an aqueous phase and an organic phase;
a second step of mixing the two-phase separated liquid with a strong alkali soluble in water so that an amount of the strong alkali is 20% or more with respect to water in the aqueous phase in terms of weight ratio to regenerate a cation of the neutralized-salt type ionic liquid present in the aqueous phase into a neutral substance and move the neutral substance from the aqueous phase to the organic phase; and
a third step of recovering the organic phase containing the neutral substance by a liquid-separating operation.

2. The recovery method according to claim 1, comprising:
a fourth step of mixing the organic phase recovered in the third step with an aqueous solution of a new neutralized-salt type ionic liquid; and
a fifth step of repeating the second step and the third step again after the fourth step to recover the organic phase in which a content of the neutral substance is increased.

3. The recovery method according to claim 1 or 2, wherein in the second step, the two-phase separated liquid and the strong alkali are mixed so that the amount of the strong alkali is 25% or more with respect to the water in terms of weight ratio.

4. The recovery method according to any one of claims 1 to 3, wherein in the second step, a reaction liquid temperature during mixing the two-phase separated liquid with the strong alkali is 20°C or lower.

5. The recovery method according to claim 4, wherein the reaction liquid temperature is 10°C or lower.

6. The recovery method according to any one of claims 1 to 5, wherein in the third step, the recovery of the organic phase is started within 1 hour from completion of mixing in the second step.

7. The recovery method according to claim 6, wherein in the third step, the recovery of the organic phase is started within 30 minutes from completion of mixing in the second step.

8. The recovery method according to any one of claims 1 to 7, wherein the strong alkali is sodium hydroxide or potassium hydroxide.

9. The recovery method according to any one of claims 1 to 8, wherein the cation of the neutralized-salt type ionic liquid has the following Formula (1) or (2).

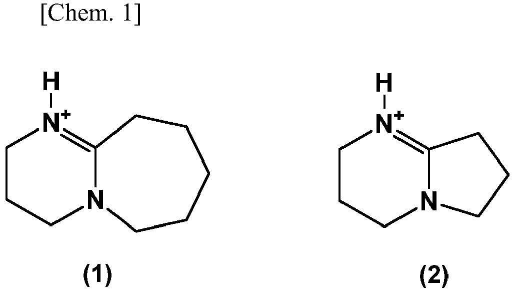

10. The recovery method according to any one of claims 1 to 9, wherein an anion of the neutralized-salt type ionic liquid is an anion of an organic acid.

11. The recovery method according to claim 10, wherein the organic acid is acetic acid.

12. The recovery method according to any one of claims 1 to 11, wherein the neutralized-salt type ionic liquid has a cellulose dissolving ability.

13. The recovery method according to claim 12, wherein the aqueous solution of the neutralized-salt type ionic liquid used in the first step is a coagulation liquid obtained after cellulose regeneration from a neutralized-salt type ionic liquid solution of cellulose.

14. A method for regenerating a neutralized-salt type ionic liquid, the regeneration method comprising mixing the organic phase containing the neutral substance recovered by the recovery method according to any one of claims 1 to 13 with an acid providing an anion component constituting the original neutralized-salt type ionic liquid and then removing the organic solvent to regenerate a neutralized-salt type ionic liquid.

15. A method for isolating a neutral substance, the isolation method comprising distilling the organic phase containing the neutral substance recovered by the recovery method according to any one of claims 1 to 13 to remove the organic solvent, and isolating the neutral substance.

16. A method for regenerating a neutralized-salt type ionic liquid, the regeneration method comprising mixing the neutral substance obtained by the isolation method according to claim 15 with an acid providing an anion component constituting the original neutralized-salt type ionic liquid to regenerate a neutralized-salt type ionic liquid.
